# EUROPÄISCHE PATENTSCHRIFT

(11) **EP 2 011 504 B1**
(45) Veröffentlichungstag und Bekanntmachung des Hinweises auf die Patenterteilung: **28.12.2011**
(21) Anmeldenummer: 08011814.4
(22) Anmeldetag: 01.07.2008
(51) Int. Cl.: A61K 33/38, A61K 45/06, A61P 17/02, A61P 17/04, A61K 36/185

(54) **Dermatikum zur Behandlung und/oder Pflege der Haut bei Neurodermitits**
Skin cream for treatment and/or cleaning of skin for neurodermitis
Produit dermatologique destiné au traitement et/ou au soin de la peau en cas de neurodermatite

(30) Priorität: 06.07.2007 DE 102007031650
(43) Veröffentlichungstag der Anmeldung: 07.01.2009
(73) Patentinhaber: STADA ARZNEIMITTEL AG, 61118 Bad Vilbel (DE)
(72) Erfinder: Hansen, Peter, Dr., 63303 Dreieich (DE); Heppner, Andrea, 61197 Florstadt (DE); Rillmann, Thomas, Dr., 76756 Bellheim (DE); Schumann, Christof, 35767 Breitscheid (DE)
(74) Vertreter: Wittkopp, Alexander

(56) Entgegenhaltungen:
- EP-A- 1 520 577
- DE-A1-102004 016 129
- DE-A1-102005 008 299
- US-A1- 2003 180 378
- US-A1- 2005 271 743
- SWARNA EKANAYAKE-MUDIYANSELAGE ET AL: "Anwendungsbeobachtung mit einem topischen silberhaltigen Pflegeprodukt (Multilind TM MikroSilber Creme) zur Überprüfung der Wirksamkeit, Verträglichkeit und kosmetischen Akzeptanz beim atopischen Ekzem" 1. Januar 2007 (2007-01-01), KOSMETISCHE MEDIZIN, GROSSE VERLAG GMBH, BERLIN, DE, PAGE(S) 291 - 295 , XP009106957 ISSN: 1430-4031 * das ganze Dokument *
- RING J ET AL: "Evening primrose oil in neurodermatitis? = Nachtkerzenöl bei Neurodermitis?" 1. September 1991 (1991-09-01), MEDIZINISCHE MONATSSCHRIFT FUER PHARMAZEUTEN, STUTTGART, DE, PAGE(S) 282 , XP009107008 ISSN: 0342-9601 * das ganze Dokument *
- HOLTMANN U: "Drug products made from Oenothera biennis oil = Nachtkerzenöl-haltige Produkte. GLS ist nicht gleich GLS" 1. Januar 1996 (1996-01-01), TW PAEDIATRIE, KARLSRUHE, DE, PAGE(S) 309,311 , XP009107007 ISSN: 0935-3216 * das ganze Dokument *

## Beschreibung

Die Erfindung betrifft eine Zusammensetzung, umfassend Silberpartikel einer mittleren Partikelgröße von 1 bis 100 µm und Nachtkerzenöl. Die Erfindung betrifft auch die Verwendung einer solchen Zusammensetzung als Hautpflegemittel, insbesondere zur Pflege trockener Hautzustände. Die Erfindung betrifft auf die Verwendung von Silberpartikeln einer mittleren Partikelgröße von 1 bis 100 µm in Kombination mit Nachtkerzenöl zur Herstellung einer pharmazeutischen Zusammensetzung zur Behandlung der Neurodermitis.

Trockene Haut sieht rau, glänzend oder rissig aus, manchmal auch gerötet oder schuppig. Bei trockener Haut können sich Falten bilden oder verstärken. Trockene Haut ist aber nicht nur ein kosmetisches Problem, denn trockene Haut kann empfindlich werden, spannen und jucken. Darüber hinaus neigt trockene Haut zu Entzündungen und kann die Entstehung von Ekzemen begünstigen.

Trockene Haut kann anlagebedingt auftreten, tritt häufig bei älteren Menschen auf, bei denen die Epidermis nicht mehr ausreichend Lipide produziert, und kann infolge übermäßiger Pflegemaßnahmen, trockener Luft sowie chronischen UV-Schäden auftreten.

Üblicherweise werden zur Pflege trockener Haut Mittel eingesetzt, die den Feuchtigkeitsverlust der Haut ausgleichen und die Oberhaut vor Austrocknung schützen. Dazu werden üblicherweise W/O-Emulsionen eingesetzt, die der Haut Feuchtigkeit und Fett zuführen sollen und die Hornschichtbarriere stabilisieren sollen. Derartige Pflegemittel weisen keinen Alkohol auf.

Die Neurodermitis ist eine Hautkrankheit, deren Hauptsymptome rote, schuppende, unter Umständen auch nässende Ekzeme auf der Haut und ein oft quälender Juckreiz sind. Weitere geläufige Bezeichnungen für die Neurodermitis sind atopisches Ekzem, atopische Dermatitis und endogenes Ekzem. Die Neurodermitis stellt eine chronische Entzündungsreaktion der Haut dar.

Die Neurodermitis tritt oft das erste Mal bei Kleinkindern auf, aber auch Jugendliche und Erwachsene können erstmalig an Neurodermitis erkranken. Die Symptome der Krankheit können sich bei verschiedenen Betroffenen in unterschiedlicher Ausprägung und an verschiedenen Hautarealen zeigen. Die Neurodermitis kann in Schüben unterschiedlicher Dauer und Stärke auftreten.

Die Neurodermitis äußert sich symptomatisch insbesondere in einer sehr empfindlichen und trockenen Haut, die oft auch gerötet ist. Ein Hautproblem stellt für die Betroffenen oftmals der starke Juckreiz dar. Durch Kratzen der Haut kann diese rissig werden, wodurch unter Umständen Ekzeme entstehen können, die oftmals nässen und den Juckreiz wiederum weiter verschlimmern. Die häufigsten Hautareale, die durch Neurodermitis betroffen sind, sind die Armbeugen, die Kniekehlen sowie die Hals- und Gesichtspartien.

Auch wenn die Ursache der Neurodermitis bislang nicht zweifelsfrei geklärt ist, wird davon ausgegangen, dass sie sowohl von genetischen Faktoren als auch von Umwelteinflüssen ausgelöst wird. Dabei wird davon ausgegangen, dass die Betroffenen aufgrund genetischer Veranlagung stärker auf bestimmte Umwelteinflüsse reagieren als andere.

Im Stand der Technik sind viele Möglichkeiten der Behandlung von Neurodermitis bekannt, zu denen neben medikamentösen Therapien auch die Vermeidung von bestimmten Nahrungsmitteln sowie äußeren Reizen oder aber Stress zählt. Als medikamentöse Therapie wird häufig die Neurodermitis durch Kortisonsalben behandelt. Bei schweren Schüben kann auch eine orale Verabreichung von kortisonhaltigen Arzneimitteln in Frage kommen. Nachteil einer Kortison-Behandlung ist aber, dass sie oft nur für kurze Zeit bei akuten Neurodermitisschüben angewendet werden kann und eine Hautverdünnung, eine starke Behaarung, Dehnungsstreifen sowie eine partielle Unterdrückung des örtlichen Immunsystems mit sich bringt. Bei längerer Anwendung kortisonhaltiger Präparate kann auch eine systemische Schädigung des Organismus auftreten, wie z. B. Leberschädigungen oder aber das Hervorrufen eines Glaukoms. Aber auch andere medikamentöse Therapien, wie z. B. eine Behandlung mit Gerbstoffpräparaten oder Schieferölpräparaten sind aus dem Stand der Technik bekannt. Nachteilig an diesen Behandlungsmethoden ist aber, dass diese Zubereitungen nicht geruchsneutral sind. Als weitere medikamentöse Behandlungstherapie steht in der Neurodermitis-Behandlung die Verabreichung von Antihistaminika zur Verfügung. Nachteil dieser Behandlung ist aber unter anderem deren müde machender Effekt. Da einige von Neurodermitis betroffene Personen allergisch auf bestimmte Lebensmittel reagieren, die die Symptome der Neurodermitis verstärken, ist auch durch Verzicht auf derartige Allergie auslösende Nahrungsmittel eine Besserung der neurodermitischen Symptome gegeben. Des weiteren besteht der Verdacht, dass Neurodermitis durch Stress verstärkt wird, so dass eine Vermeidung von Stresssituationen durchaus eine Linderung der neurodermitischen Probleme mit sich bringen kann. Insbesondere wurde ein Einfluss von Stress auf den neurodermitischen Hautzustand bei Kindern beobachtet. Es sind auch die Behandlung der Neurodermitis durch Klimawechsel und physikalische Therapien bekannt. Dazu zählen z. B. die Behandlung der Neurodermitis durch längeren Aufenthalt in Gebirgen oder aber das Baden in Salzwasser, bevorzugt an der Nordsee. Generell scheinen Gegenden mit jodhaltiger Luft empfehlenswert. Auch die Bestrahlung mit hochdosiertem UV-Licht stellt eine Therapiemöglichkeit der Neurodermitis dar. Nachteil dieser Therapieverfahren ist ganz offensichtlich der mit ihrer Durchführung verbundene Umstand, wie z. B. ein spezieller Kuraufenthalt in anderen geographischen Gegenden.

Eine Aufgabe der vorliegenden Erfindung ist es deshalb unter anderem, eine Behandlung der Neurodermitis anzugeben, die die Nachteile der herkömmlichen Behandlungsverfahren der Neurodermitis überwindet. Sie soll einfach anwendbar sein und nicht die von bisher bekannten beispielsweise medikamentösen Therapien bekannten Nachteile mit sich bringen. Diese Aufgabe wird durch die Verwendung von Silberpartikeln einer mittleren Partikelgröße von 1 µm bis 100 µm in Kombination mit Nachtkerzenöl zur Behandlung der Neurodermitis bzw. zur Herstellung eines Heilmittels zur Behandlung der Neurodermitis gelöst.

Eine weitere Aufgabe der vorliegenden Erfindung besteht darin, eine gegenüber dem Stand der Technik verbesserte Behandlung von trockener Haut anzugeben und ein dafür geeignetes Dermatikum bereitzustellen. Diese Aufgabe wird durch Verwendung von Silberpartikeln einer mittleren Partikelgröße von 1 µm bis 100 µm in Kombination mit Nachtkerzenöl als Dermatikum bei trockener Haut, insbesondere neurodermitischen Hautzuständen gelöst.

Gemäß einer Ausführungsform der Erfindung werden Silberpartikel mit einer mittleren Partikelgröße von 1 bis 100 µm in Kombination mit Nachtkerzenöl zur Behandlung der Neurodermitis bzw. zur Herstellung eines Medikamentes zur Behandlung der Neurodermitis verwendet.

Im Stand der Technik ist die antimikrobielle Wirkung metallischen Silbers hinreichend bekannt (US 2005/0271743 A1, US 2003/4180378 A1,

DE 10 2005 008 299 A1). Erfindungsgemäß werden nun aber Silberpartikel einer Größe von 1 µm bis 100 µm, sogenanntes Mikrosilber oder Microsilver,in Kombination mit Nachtkerzenöl zur Behandlung der Neurodermitis eingesetzt. Überraschend wurde gefunden, dass Silberpartikel einer mittleren Partikelgröße von 1 µm bis 100 µm einen positiven Einfluss auf die unter dem Krankheitsbild der Neurodermitis auftretenden Beschwerden wie Ödem/Papeln-Hildung, Rötung/Erythem-Bildung, Krustenbildung, Excoriation, Lichenifikation, verstärktem Juckreiz, Trockenheit (Xerosis), Schuppung sowie Schlafmangel haben. Es wurde gefunden, dass die topische Anwendung einer Zusammensetzung, die Silberpartikel einer mittleren Partikelgröße von 1 µm bis 100 µm sowie Nachtkerzenöl umfasst, einen positiven Einfluss auf die unter Neurodermitis auftretenden Probleme hat. In klinischen Untersuchungen wurde gefunden, dass der Atopie-Index von unter Neurodermitis leidenden Personen unter topischer Behandlung mit einer Mikrosilber und Nachtkerzenöl enthaltenden Zusammensetzung abnahm, in vielen Fällen die neurodermitischen Probleme sogar vollständig verschwanden.

Aus dem Stand der Technik ist kolloidales Silber bekannt. Kolloidales Silber besteht aus elektrisch geladenen Silberteilchen in Wasser. Die Partikelgröße des Silbers im kolloidalen Silber liegt zwischen 1 und 10 nm. Bei topischer Anwendung dieses kolloidalen Silbers können die Silberpartikel in tiefere Hautschichten eindringen. Die Auswirkungen der Silber-Nanopartikel auf die menschliche Gesundheit, wenn sie in den Körper eingedrungen sind, sind bisher nicht bekannt. Es ist aber bekannt, dass sich nanoskaliertes Silber, sogenanntes kolloidales Silber, bei zu hohen Konzentrationen im Körper ablagern kann und zu sogenannten Silberablagerungen führen kann.

Die erfindungsgemäß verwendeten Silberpartikel einer Größe von 1 µm bis 100 µm weisen nicht die mit der Verwendung von Silber-Nanopartikeln einer Größe von 1 bis 100 nm verbundenen Nachteile auf. Die erfindungsgemäß verwendeten Silberpartikel sind derart groß, dass sie bei topischer Anwendung nicht in den menschlichen Körper eindringen können. Sie können nicht über die Blutbahn in den Körper aufgenommen werden.

Die Silberpartikel gemäß vorliegender Erfindung weisen eine mittlere Partikelgröße von 1 µm bis 100 µm auf, vorzugsweise 2 µm bis 20 µm und besonders bevorzugt 2 µm bis 5 µm. Es wird nach im Stand der Technik bekannten Verfahren hergestellt. Dazu zählen beispielsweise das mechanische Zermahlen in Kolloidmühlen sowie die elektrolytische Herstellung über verschiedene Verfahren wie z. B. mit Umkehrosmosewasser oder dampfdestilliertem Wasser in erwärmtem Zustand. Auch rein chemische Verfahren zur Herstellung von Silberpartikeln einer Größe von 1 bis 100 µm über Reduktion von Silbersalzen sind anwendbar.

Die erfindungsgemäßen Silberpartikel bestehen vorzugsweise aus reinem Silber. Sie können aber auch in einem geringen Anteil von bis zu 1 Gew.-% andere Metalle enthalten, wie z. B. Zink und/oder Kupfer. Besonders vorteilhaft weisen die Silberpartikel Verunreinigungen von weniger als 5 ppm auf, besonders vorteilhaft bestehen sie aus reinem Silber. Mögliche Verunreinigungen der Silberpartikel können z. B. Kalium, Natrium oder Chlor sein.

Gemäß einer erfindungsgemäßen Ausführungsform liegen die Silberpartikel in einer mittleren Teilchengröße von 1 µm bis 100 µm als poröse Partikel vor. Die mittlere innere Porosität der Silberpartikel kann z. B. mindestens 65 %, insbesondere 65 bis 95 % betragen. Besonders bevorzugt ist eine mittlere innere Porosität von 85 % bis 95 %. Die Porosität der Teilchen kann gemäß dem in WO 2005/023213 auf Seite 7 beschriebenen Verfahren bestimmt werden.

Gemäß einer erfindungsgemäßen Ausführungsform liegen die verwendeten Silberpartikel als Agglomerate metallischer Primärpartikel vor, deren mittlerer Partikeldurchmesser zwischen 10 und 200 nm, vorzugsweise zwischen 15 und 80 nm liegt. Derartige poröse Silberpartikel können gemäß dem in WO 2005/023213 beschriebenen Verfahren hergestellt werden, insbesondere dem auf Seite 10 von WO 2005/023213 beschriebenen Verfahren. Derartige Silberpartikel mit einem mittleren Partikeldurchmesser von 1 µm bis 100 µm, die eine innere Porosität aufweisen, sind z. B. von der Firma Biogate erhältlich.

Gemäß einer weiteren bevorzugten Ausführungsform der Erfindung wird eine Kombination der beschriebenen Silberpartikeln mit einer mittleren Partikelgröße von 1 bis 100 µm und Nachtkerzenöl zur Behandlung der Neurodermitis bzw, zur Herstellung eines Medikaments zur Behandlung der Neurodermitis verwendet.

Die Kombination von Nachtkerzenöl und Osmolyten X aus extremophilen Mikroorganismen zur Pflege sehr trockener, irritierter, schuppiger und problematischer Haut ist aus DE 10 2004 016 129 A1 bekannt. Aus EP 1 520 577 A1 ist die Kombination von Nachtkerzenöl und unverseifbaren Bestandteilen eines weiteren pflanzlichen Öls in einem synergistischen Verhältnis bekannt, sowie deren Verwendung zur Behandlung trockener Haut.

Es wurde nun überraschend gefunden, dass Nachtkerzenöl in Kombination mit Silberpartikeln einer mittleren Partikel größe von 1 bis 100 µm in der Behandlung der Neurodermitis bzw. zur Herstellung eines Medikaments zur Behandlung der Neurodermitis verwendet werden kann.

Die Behandlung der Neurodermitis mit einer Kombination aus Silberpartikeln einer mittleren Partikelgröße von 1 bis 100 µm und Nachtkerzenöl kann sowohl eine pharmazeutische als auch eine kosmetische sein und erfolgt durch topisches Auftragen der Silberpartikel in Kombination mit Nachtkerzenöl auf die von Neurodermitis betroffenen Hautstellen.

Ebenso wird gemäß einer weiteren Ausführungsform der Erfindung eine Kombination der beschriebenen Silberpartikeln mit einer mittleren Partikelgröße von 1-100 µm und Nachtkerzenöl zur Pflege trockener Haut, insbesondere neurodermitischer Hautzustände verwendet bzw. zur Herstellung eines Heilmittels zur Pflege und/oder Behandlung trockener Haut, insbesondere neurodermitischer Hautzustände.

Ein weiterer Gegenstand der vorliegenden Erfindung sind stabile Zusammensetzungen, die die beschriebenen Silberpartikel einer mittleren Partikelgröße von 1 bis 100 µm sowie Nachtkerzenöl umfassen.

Es war aus dem Stand der Technik bekannt, dass Nachtkerzenöl eine große Menge ungesättigter Fettsäuren, insbesondere einen großen Anteil an cis-Linolsäure, enthält. Es ist damit sehr anfällig gegenüber Verderb, insbesondere oxidativem Verderb, der durch die Gegenwart von katalytisch wirkenden Metallen in Nachtkerzenöl enthaltenden Zusammensetzungen beschleunigt wird.

Es wurde nun überraschend gefunden, dass durch Kombination von Silberpartikeln einer mittleren Partikelgröße von 1 µm bis 100 µm und Nachtkerzenöl dennoch stabile Zusammensetzungen erhalten werden.

So wurde des weiteren überraschend gefunden, dass die Verwendung dieser erfindungsgemäßen Zusammensetzungen einen positiven Effekt in der Behandlung trockener Haut, insbesondere neurodermitischer Hautzustände und der Neurodermitis hat. Insbesondere wurde gefunden, dass die Verwendung der beschriebenen Silberpartikeln einer mittleren Partikelgröße von 1 bis 100 µm und Nachtkerzenöl, die unter dem Krankheitsbild der Neurodermitis auftretenden Beschwerden wie Ödem/Papeln-Bildung, Rötung/Erythem-Bildung, Krustenbildung, Excoriation, Lichenifikation, verstärktem Juckreiz, Trockenheit (Xerosis), Schuppung sowie Schlafmangel mindern bzw. verschwinden lassen. Es wurde gefunden, dass die topische Anwendung einer sowohl die beschriebenen Silberpartikeln einer mittleren Partikelgröße von 1 bis 100 µm sowie Nachtkerzenöl enthaltenden Zusammensetzung erfolgreich in der Pflege trockener Haut, insbesondere neurodermitischer Hautzustände, und der Behandlung der Neurodermitis eingesetzt werden kann. Ihre Anwendung führt zu einer Minderung der chronischen Entzündungsreaktion der Haut.

Die erfindungsgemäßen Zusammensetzungen bzw. Heilmittel, enthaltend die beschriebenen Silberpartikeln und Nachkerzenöl, liegen gemäß einer bevorzugten Ausführungsform als O/W-Emulsion vor, insbesondere als alkoholische O/W-Emulsion. Aus dem Stand der Technik bekannte Pflegemittel für trockene Haut und Mittel zur Behandlung der Neurodermitis sind als W/O-Emulsion formuliert, damit die Ölphase im direkten Hautkontakt steht. Ebenso sind die im Stand der Technik bekannten Pflegeprodukte für trockene Haut, insbesondere neurodermitische Hautzustände, ebenso wie die Heilmittel zur Behandlung der Neurodermitis alkoholfrei, da Alkohol die von Neurodermitis betroffene Haut austrocknet.

Überraschenderweise wurde gefunden, dass in den erfindungsgemäßen, die beschriebenen Silberpartikeln und Nachtkerzenöl enthaltenden, Zusammensetzungen, die eine O/W-Galenik aufweisen, der darin enthaltende Alkohol nicht zu einer weiteren Austrocknung der Haut oder Verschlimmerung der unter dem Krankheitsbild der Neurodermitis auftretenden Beschwerden führt, sondern nebst der mikrobiologischen Wirkung einen angenehm kühlenden Effekt hat und vollständig hautverträglich ist. Die erfindungsgemäßen Zusammensetzungen zeichnen sich gemäß einer bevorzugten Ausführungsform durch einen Alkoholgehalt von mindestens 1 Gew.-%, insbesondere 5-15 Gew.-% aus.

Die erfindungsgemäßen Zusammensetzungen zeichnen sich gemäß einer weiteren bevorzugten Ausführungsform dadurch aus, dass sie frei von PEG-Emulgatoren, das heißt Emulgatoren auf Polyethylenglykol-Basis, frei von Konservierungsmitteln und frei von Parfümen sind.

Die erfindungsgemäßen Zusammensetzungen werden als stabile Silberpartikelformulierungen, beispielsweise als Lotion, Pasten, Salbe, Creme, Emulsion, Gel, Puder oder Suspension formuliert.

Stabil bedeutet, dass die Formulierung lagerstabil ist, dass heißt die Silberpartikel bei Lagerung nicht sedimentieren und ggf. enthaltenes Nachtkerzenöl nicht silberkatalytisch zersetzt wird.

Die Silberpartikel liegen in den erfindungsgemäßen Zusammensetzungen in Mengen von 0,001 Gew.-% - 10 Gew.-%, bevorzugt von 0,01 Gew.-% - 5 Gew.-% und besonders bevorzugt von 0,1 Gew.-% - 1 Gew.-% sowie insbesondere 0,1 Gew.-% - 0,5 Gew.-% vor und werden auch gemäß der beanspruchten Verwendung bevorzugt in diesen Mengenbereichen verwendet.

Das Nachtkerzenöl liegt in den erfindungsgemäßen Zusammensetzungen in Mengen von 0,1 Gew.-% - 30 Gew.-%, besonders bevorzugt von 1 Gew.-% - 10 Gew.-% vor und wird gemäß der weiteren beanspruchten Verwendung ebenfalls bevorzugt in diesem Mengenbereich angewendet.

Von der Anmelderin im Rahmen einer Studie durchgeführte Versuche mit verschiedenen Zusammensetzungen, umfassend Silberpartikel einer mittleren Partikelgröße von 1 bis 100 µm und Nachtkerzenöl haben eine hochsignifikante Verringerung der mit einer Neurodermitis verbundenen Beschwerden wie Ödem/Papeln-Bildung, Rötung/Erythem-Bildung, Krustenbildung, Excoriation, Lichenifikation, verstärktem Juckreiz, Trockenheit (Xerosis) sowie Schlafmangel ergeben. Es wurde gefunden, dass die topische Anwendung einer Zusammensetzung, umfassend Silberpartikel mit einer mittleren Partikelgröße von 1 bis 100 µm sowie Nachtkerzenöl den Atopie-Index von unter Neurodermitis leidenden Personen bei topischer Anwendung dieser Zusammensetzung senkt und in vielen Fällen die neurodermitischen Probleme sogar behebt.

Die folgenden Beispiele erfindungsgemäßer Zusammensetzungen und im Rahmen einer Studie durchgeführter Versuche sollen die vorliegende Erfindung verdeutlichen, ohne sie einzuschränken.

### Beispiele 1-5: Cremes

| **Inhaltsstoff (INCI)** | **Beispiel 1** | **Beispiel 2** | **Beispiel 3** | **Beispiel 4** | **Bespiel 5** |
|---|---|---|---|---|---|
| Polyglyceryl-3-Methylglucose Distearate | 4,00 | 4,0 | 4,2 | 3,8 | 4,2 |
| Hydrogenated Coco-Glycerides | 1,00 | 1,00 | 0,8 | 1,1 | 0,8 |
| Cetyl Palmitate | 1,00 | 0,9 | 0,8 | 1,2 | 0,9 |
| Cetyl Alcohol | 3,00 | 2,5 | 2,5 | 3,2 | 3 |
| C12-15 Alkyl Benzoate | 8,00 | 7,8 | 7,8 | 8,5 | 8,2 |
| Simmondsia Chinensis Seed Oil | 6,00 | 5,5 | 5,5 | | 6 |
| Isopropylmyristate | 5,00 | 4,5 | 4,5 | 5 | 5 |
| Octyldodecanol, Echium Plantagineum, Cardiospermum, Halicacabum, Helianthus Annuus | 2,00 | 1 | 1 | 0 | 0 |
| Nachtkerzenöl | 6,00 | 4,00 | 2,00 | 8,00 | 5,00 |
| Tocopheryl Acetate | 1,00 | 0,8 | 1,2 | 1,2 | 1 |
| Microsilver | 0,30 | 0,32 | 0,5 | 0,25 | 0,1 |
| Aqua | 46,40 | 53,9 | 62,14 | 45,91 | 55,28 |
| Xanthan Gum | 0,30 | 0,28 | 0,26 | 0,34 | 0,32 |
| Glycerin 99,5 % | 4,00 | 3,5 | 3,8 | 4,5 | 4,2 |
| Alcohol Denat. | 12,00 | 10,00 | 3,00 | 11,00 | 0 |
| Butylenglykol | 0 | 0 | 0 | 0 | 6 |

Zur Herstellung der O/W-Emulsionen gemäß der Beispiele 1-5 wurde zu der auf 70 °C erwärmten und homogenisierten Phase I, bestehend aus Polyglyceryl-3-Methylglucose Distearate, Hydrogenated Coco-Glycerides, Cetyl Palmitate, Cetyl Alcohol, C12-15 Alkyl Benzoate, Simmondsia Chinensis Seed Oil, Isopropylmyristate, Nachtkerzenöl, Tocopheryl Acetat und Microsilver, sowie gegebenenfalls Octyldodecanol, Echium Plantagineum, Cardiospermnum, Halicacabum und Helianthus Annuus, unter Rühren die auf 75 °C erwärmte Phase II, bestehend aus Aqua, Xanthan Gum, Glycerin und gegebenenfalls Butylenglykol zugegeben. Die Zusammensetzung wird weiter homogenisiert, anschließend auf 45 °C abgekühlt und gegebenenfalls Alcohol Denat, zugegeben. Nach weiterem Homogenisieren wird die Masse schließlich unter Rühren auf Raumtemperatur abgekühlt.

### Beispiele 6-10: Lotionen

| **Inhaltsstoff (INCI)** | **Beispiel 6 Einwaage [g/100g]** | **Beispiele 7 Einwaage [g/100g]** | **Beispiele 8 Einwaage [g/100g]** | **Beispiel 9 Einwaage [g/100g]** | **Beispiel 10 Einwaage [g/100g]** |
|---|---|---|---|---|---|
| Polyglyceryl-3-Methylglucose Distearate | 3,00 | 3,00 | 3,2 | 2,8 | 2,8 |
| Hydrogenated Coco-Glycerides | 0,75 | 0,75 | 0,7 | 0,8 | 0,8 |
| Cetyl Palmitate | 0,75 | 0,75 | 0,9 | 0,8 | 0,7 |
| Ceryl Alcohol | 2,25 | 2,25 | 2,5 | 2,2 | 2,0 |
| C12-15 Alkyl Benzoate | 8,00 | 8,00 | 7,8 | 8,5 | 8,2 |
| Simmondsia Chinensis Seed Oil | 6,00 | 6,00 | 4,00 | 7,00 | 5,00 |
| Isopropylmyristate | 5,00 | 5,00 | 5,5 | 5,2 | 4,8 |
| Octyldodecane, Echium Palntagineum, Cardiospernum, Halicacabum, Helianthus, Annus | 2,00 | 2,00 | 2,5 | 1 | 0 |
| Nachtkerzenöl | 6,00 | 6,00 | 4,00 | 2,00 | 8,00 |
| Tocopheryl Acetate | 1,00 | 1,00 | 0,8 | 1,2 | 1,5 |
| Microsilver | 0,20 | 0,10 | 0,25 | 0,15 | 0,5 |
| Aqua | 48,75 | 48,85 | 53,27 | 50,65 | 56,25 |
| Xanthan Gum | 0,30 | 0,30 | 0,32 | 0,2 | 0,25 |
| Glycerin | 4,00 | 4,00 | 3,8 | 3,5 | 4,2 |
| Alcohol Denat. | 12,00 | 12,00 | 3,00 | 14,00 | 0 |
| Butylenglykol | 0 | 0 | 0 | 0 | 5,00 |

| | | | | | |
|---|---|---|---|---|---|
| Auch diese Lotionen werden entsprechend der Herstellungsvorschrift zu den Beispielen 1-5 hergestellt. | | | | | |

Es wurde eine Studie an zwei Probandenkollektiven A und B ä 15 erwachsenen Testpersonen mit leichten Hautveränderungen der Neurodermitis, wie Juckreiz, Rötung, Schuppung und Trockenheit durchgeführt. Es durfte keines der folgenden Ausschlusskriterien vorliegen: akute organische Krankheit, Schwangerschaft und Stillzeit, bestehende Sensibilisierung auf Inhaltsstoffe der Prüfprodukte, schwerwiegende Erkrankungen, Applikation von wirkstoffhaltigen Präparaten und Pflegemitteln bis vier Wochen vor Testbeginn sowie die Einnahme von Medikamenten gegen Neurodermitis/Atopie, wie Glucocorticoide, Antiallergika, topische Immunmodulatoren, etc.

In folgender Tabelle ist eine Liste der Teilnehmer des Probandenkollektivs A angegeben:

**Tabelle 1: Probanden des Probandenkollektivs A**

| **Nummer** | **Geschlecht** | **Alter** | **Diagnose** | **Applikationsort** |
|---|---|---|---|---|
| 1 | w | | Neurodermitis | Arme, Beine |
| 2 | w | 42 | Neurodermitis | Arme, Handrücken |
| 3 | w | 50 | Neurodermitis | Arme, Beine |
| 4 | w | 44 | Neurodermitis | Arme, Beine |
| 5 | w | 47 | Neurodermitis | Arme, Beine |
| 6 | w | 18 | Neurodermitis | Arme, Beine |
| 7 | w | 44 | Neurodermitis | Stirn, Arme |
| 8 | w | 42 | Neurodermitis | Arme, Handrücken |
| 9 | m | 43 | Neurodermitis | Beine |
| 10 | m | 40 | Neurodermitis | Arme, Handrücken |
| 11 | m | 46 | Neurodermitis | Arme |
| 12 | w | 28 | Neurodermitis | Arme |
| 13 | w | 38 | Neurodermitis | Arme, Handrücken |
| 14 | w | 36 | Neurodermitis | Arme, Handrücken |
| 15 | w | 66 | Neurodermitis | Arme, Handrücken |

Im Folgenden ist eine Liste der Teilnehmer des Probandenkollektivs B angegeben:

**Tabelle 2: Probanden des Probandenkollektivs B**

| **Nummer** | **Geschlecht** | **Alter** | **Diagnose** | **Applikationsort** |
|---|---|---|---|---|
| 1 | w | 34 | Neurodermitis | Gesicht, Arme und Beine |
| 2 | w | 20 | Neurodermitis | Arme |
| 3 | w | 19 | Neurodermitis | Arme, Handrücken |
| 4 | w | 45 | Neurodermitis | Arme, Beine |
| 5 | m | 34 | Neurodermitis | Arme, Handrücken |
| 6 | w | 55 | Neurodermitis | Arme |
| 7 | w | 50 | Neurodermitis | Arme, Beine |
| 8 | w | 19 | Neurodermitis | Arme, Beine |
| 9 | w | 34 | Neurodermitis | Arme, Beine |
| 10 | w | 39 | Neurodermitis | Arme, Beine, Handrücken |
| 11 | w | 49 | Neurodermitis | Arme, Beine |
| 12 | w | 46 | Neurodermitis | Arme, Beine |
| 13 | m | 35 | Neurodermitis | Gesicht, Arme, Beine |
| 14 | w | 44 | Neurodermitis | Arme, Beine |
| 15 | w | 36 | Neurodermitis | Beine |

Zu Beginn der Studie wurden den Probanden der Kollektive A und B das Produkt Creme gemäß Beispiel 1 sowie die Probandentagebücher mit der Applikationsinstruktion über mindestens 2x-tägliche Applikation ausgehändigt. Weiterhin wurden die Probanden gebeten, in der Zeit des Anwendungstests auf andere Präparate im Testarial zu verzichten.

Nach zehn Tagen wurde ein erneuter Status erhoben. Dabei wurde erneut der ärztliche Beurteilungsbogen, sowie der Fragebogen für das Produkt erhoben. Weiterhin wurde um Rückgabe der Probandentagebücher gebeten.

Danach erfolgte im Probandenkollektiv A der Produktwechsel zur Lotion gemäß Beispiel 6 und im Probandenkollektiv B der Wechsel zur Lotion gemäß Beispiel 7. Diese Lotionen wurden für 18 Tage unter 2x-täglicher Applikation angewandt. Nach der Anwendungsphase wurde erneut der atopische Index innerhalb der dermatologischen Abschlussuntersuchung erhoben sowie die Probandentagebücher zurückgefordert.

Der in der Studie ermittelte Atopie-Index diente der qualitativen und quantitativen Einschätzung des Schweregrades des atopischen Ekzems, modifizierter SCORAD (= Severity Scoring of Atopic Dermatitis). Die standardisierte Beurteilung erfolgt durch die Angabe;
A) des Ausprägungsgrades sechs typischer morphologischer Veränderungen, nämlich: Hautrötung, Krusten, flächenhafte Infiltration der Haut mit Vergröberungen der Hautfelder, Schwellung/Bläschen (hier: Ausschlusskriterium), Hautabschürfungen bis ins Korium (hier: Ausschlusskriterium), Trockenheit (wird an den nicht betroffenen Hautstellen erhoben). Der Ausprägungsgrad reicht von 0-3, wobei 0 = nicht vorhanden, 1 = milde vorhanden, 2 = mittelgradig und 3 = ausgeprägt bedeuten. Die Summe der Werte kann zwischen 0 und 10 schwanken, wobei die Ausprägung von Ödem oder Papeln als Ausschlusskriterium definiert wurde, als Anhalt für einen akuten neurodermitischen Hautzustand mit medizinischer Behandlungsbedürftigkeit. Eine Hautabschürfung bis in Korium wurde als Ausschlusskriterium im Rahmen der Eingangsuntersuchung definiert. Excoriationen im Verlauf der Studie waren Indikation für eine Testunterbrechung von zwei Tagen.
B) des Anteils der betroffenen Hautfläche (%) bezüglich Rötung/Kruste/Excoriation. Überschritt der Gesamtwert des SCORAD 50 aufgrund einer großflächigen Ausprägung führt dies ebenfalls zum Ausschluss des Probanden.
C) der subjektiven Einschätzung von Juckreiz anhand einer visuellen Analogskala von 0-10, außerdem des nächtlichen Schlafverlustes von 0-10. Die Einschätzung des Juckreizes > 7 und des Schlafverlustes > 3 führt ebenfalls zum Ausschluss, da medizinische Behandlungsbedürftigkeit, eventuell Artefakte anzunehmen sind.

Der maximale Gesamtwert als modifizierter SCORAD ist 50, als Ausdruck einer mittleren Ausprägung des atopischen Krankheitsbildes. Berechnung des Index: 7A/2+B/5+C.

Die begleitend zur Studie durchgeführten dermatologischen Untersuchungen ergaben, dass vor Beginn des Anwendungstests alle 30 Testpersonen entweder leichte Rötung, Schuppung oder Trockenheit zeigten und weitere pathologische Hautveränderungen in keiner Form feststellbar war. Auch im Verlauf des vierwöchigen Anwendungstests zeigte keine der 30 Testpersonen pathologische Hautveränderungen. Testunterbrechungen oder gar hautfachärztliche Behandlungen waren nicht notwendig. Ebenso zeigten sich bei der dermatologischen Abschlussuntersuchung nach dem Testende bei den 30 Testpersonen keine zusätzlichen pathologischen Hautveränderungen. Das genannte Präparat wurde sehr gut vertragen und führte bei keiner Testperson zu unerwünschten Hautveränderungen.

Die durch den modifizierten SCORAD erfassten dermatologischen Beurteilungskriterien waren:
1. Ödem/Papeln
2. Rötung/Erythem
3. Krustenbildung
4. Excoriation
5. Lichenifikation
6. Trockenheit (Xerosis)
7. Verstärkter Juckreiz
8. Schlafverlust

Weitere subjektive Beurteilungskriterien waren:
1. Schweregrad der Erkrankung
2. Rötung/Erythem
3. Trockenheit
4. Juckreiz
5. Wirkung des Prüfpräparates.

Die Bewertung des subjektiven Beurteilungskriterien erfolgte durch die Probanden im Rahmen der täglichen Dokumentation im Probandentagebuch. Die Skala umfasst den Bereich 0-7, wobei 0 = nicht vorhanden und 7 = sehr stark vorhanden bedeutet.

In den Tabellen 3-7 sind die Auswertungen der subjektive Beurteilungskriterien der Probandentagebücher des Probandenkollektivs A zusammengefasst, die nach einer zehntägigen Anwendung der Creme gemäß Beispiel 1 im 18-tägigen Zeitraum der Anwendung der Lotion gemäß Beispiel 2 von Tag 11 bis Tag 28 angefertigt wurden.

Tabelle 3 gibt den subjektiv beurteilten Schweregrad der Hauterkrankung wieder.

Tabelle 4 gibt die subjektiv beurteilte Rötung der Haut wieder.

Tabelle 5 gibt die subjektiv beurteilte Trockenheit der Haut wieder.

Tabelle 6 gibt den subjektiv beurteilten Juckreiz wieder.

Tabelle 7 gibt die subjektiv beurteilte Wirkung des Prüfpräparates (hier der Lotion gemäß Beispiel 2) wieder.

In den Tabellen 8-12 sind die Auswertungen der subjektiven Beurteilungskriterien der Probandentagebücher des Probandenkollektivs B zusammengefasst, die nach einer zehntägigen Anwendung der Creme gemäß Beispiel 1 in 18-tägigem Zeitraum der Anwendung der Lotion gemäß Beispiel 3 von Tag 11 bis Tag 28 angefertigt wurde.

Tabelle 8 gibt den subjektiv beurteilten Schweregrad der Hauterkrankung wieder.

Tabelle 9 gibt die subjektiv beurteilte Rötung wieder.

Tabelle 10 gibt die subjektiv beurteilte Trockenheit wieder.

Tabelle 11 gibt den subjektiv beurteilten Juckreiz wieder.

Tabelle 12 gibt die subjektiv beurteilte Wirkung des Prüfpräparates (hier der Lotion gemäß Beispiel 3) wieder.

In Tabelle 13 ist der Atopie-Score des Probandenkollektivs A vor Anwendung der Lotion gemäß Beispiel 6 am Tage 11 der Studie ("Eingangswert") und nach 18-tägiger Anwendung der Lotion gemäß Beispiel 6 am Tag 28 der Studie ("nach 18 Tagen") sowie die entsprechende absolute und prozentuale Veränderung angegeben.

Danach ergab sich nach einem 18-tägigen Anwendungszeitraum eine Reduktion des durchschnittlichen Atopie-Wertes von 27,14 auf 6,39. Dies entspricht einer Verringerung von - 76,44 % zum Ausgangswert.

In Tabelle 14 ist der Atopie-Score des Probandenkollektivs B vor Anwendung der Lotion gemäß Beispiel 7 am Tage 11 der Studie ("Eingangswert") und nach 18-tägiger Anwendung der Lotion gemäß Beispiel 7 am Tag 28 der Studie ("nach 18 Tagen") sowie die entsprechende absolute und prozentuale Veränderung angegeben.

Danach ergab sich nach einem 18-tägigen Anwendungszeitraum eine Reduktion des durchschnittlichen Atopie-Wertes von 19,11 auf 6,99. Dies entspricht einer Verringerung von - 63,45 % zum Ausgangswertes.

In Tabelle 15 ist der Atopie-Score der Probandenkollektive A (Probandennummer 1-15) und B (Probandennummer 16-30) vor Studienbeginn ("Eingangswert") und nach Studienende, dass heißt nach 10-tägiger Anwendung der Creme gemäß Beispiel 1 und 18-tägiger Anwendung der Lotion gemäß Beispiel 6 im Probandenkollektiv A bzw. der Lotion gemäß Beispiel 7 im Probandenkollektiv B wiedergegeben.

Es kann aus dermatologischer Sicht bezüglich dieser Anwendungsstudie an insgesamt 30 Probanden festgestellt werden, dass das zu prüfende Produktsystem nach insgesamt 4-wöchiger Anwendung unter dermatologisch klinischen Kriterien sehr gut vertragen wurde.

Der Index für Atopie wies verminderte Werte auf:

Nach zehn Tagen Anwendung an 30 Probanden der Creme gemäß Beispiel 1 sank der Atopie-Score durchschnittlich um 39,83 %.

Nach 18 Tagen Anwendung der Lotion gemäß Beispiel 6 an 15 Probanden des Kollektivs A war der Atopie-Score durchschnittlich um 76,44 % verringert.

Nach 18 Tagen Anwendung der Lotion gemäß Beispiel 7 an 15 Probanden des Kollektivs B war der Atopie-Score durchschnittlich im 63,45 % verringert.

Nach 28 Tagen Anwendung des Produktsystems aus Creme und Lotion verringertes sich der Atopie-Score durchschnittlich um 82,59 %.

Ein weiterer Gegenstand der vorliegenden Erfindung ist daher die Verwendung einer Zusammensetzung, umfassend die beschriebenen Silberpartikeln mit einer mittleren Partikelgröße von 1-100 µm und Nachtkerzenöl, insbesondere vorliegend als alkoholische O/W-Emulsion, zur Reduktion des durchschnittlichen Atopie-Scores, insbesondere bei 18- bzw. 28-tägiger Anwendung, um mindestens 40 %, bevorzugt 60 %, besonders bevorzugt 70 % und insbesondere 80 %.

**Tabelle 3: Probandenkollektiv A, Schweregrad der Hauterkrankung, Anwendung Lotion gemäß Beispiel 6**

| **Ein weiterer** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 3 | 3 | 3 | 3 | 2 | 2 | 2 | 2 | 2 | 2 | 2 | 1 | 1 | 1 | 1 | 1 | 1 | 1 |
| **2** | 4 | 3 | 3 | 3 | 3 | 3 | 3 | 3 | 2 | 2 | 2 | 2 | 2 | 2 | 2 | 1 | 1 | 1 |
| **3** | 3 | 3 | 3 | 3 | 2 | 2 | 2 | 2 | 2 | 2 | 1 | 1 | 1 | 1 | 1 | 1 | 1 | 1 |
| **4** | 4 | 3 | 3 | 3 | 3 | 3 | 3 | 2 | 2 | 2 | 2 | 2 | 1 | 1 | 1 | 1 | 1 | 1 |
| **5** | 3 | 3 | 3 | 3 | 3 | 3 | 3 | 3 | 3 | 3 | 3 | 2 | 2 | 2 | 2 | 2 | 2 | 2 |
| **6** | 2 | 2 | 2 | 2 | 2 | 2 | 2 | 2 | 2 | 2 | 2 | 2 | 2 | 2 | 2 | 2 | 2 | 2 |
| **7** | 4 | 4 | 4 | 4 | 3 | 3 | 3 | 3 | 3 | 3 | 3 | 2 | 2 | 2 | 1 | 1 | 1 | 1 |
| **8** | 3 | 3 | 3 | 3 | 3 | 3 | 2 | 2 | 2 | 2 | 2 | 2 | 1 | 1 | 1 | 1 | 1 | 1 |
| **9** | 3 | 3 | 3 | 3 | 3 | 2 | 2 | 2 | 2 | 2 | 2 | 2 | 2 | 2 | 1 | 1 | 1 | 1 |
| **10** | 3 | 2 | 2 | 2 | 2 | 2 | 2 | 2 | 1 | 1 | 1 | 1 | 1 | 1 | 1 | 1 | 1 | 1 |
| **11** | 3 | 2 | 2 | 2 | 2 | 2 | 2 | 1 | 1 | 1 | 1 | 1 | 1 | 1 | 1 | 1 | 1 | 1 |
| **12** | 3 | 3 | 3 | 3 | 3 | 3 | 3 | 3 | 3 | 3 | 3 | 3 | 2 | 2 | 2 | 2 | 2 | 1 |
| **13** | 5 | 4 | 3 | 3 | 3 | 3 | 3 | 3 | 3 | 2 | 2 | 2 | 2 | 1 | 1 | 1 | 1 | 1 |
| **14** | 2 | 2 | 2 | 2 | 2 | 2 | 2 | 2 | 2 | 2 | 2 | 2 | 2 | 2 | 2 | 2 | 2 | 2 |
| **15** | 6 | 6 | 5 | 5 | 5 | 5 | 5 | 5 | 5 | 5 | 5 | 5 | 5 | 5 | 5 | 5 | 5 | 5 |
| **Mittelwert** | 3,4 | 3,1 | 2,9 | 2,9 | 2,7 | 2,7 | 2,6 | 2,5 | 2,3 | 2,3 | 2,2 | 2,0 | 1,8 | 1,7 | 1,6 | 1,5 | 1,5 | 1,5 |
| **Min** | 2 | 2 | 2 | 2 | 2 | 2 | 2 | 1 | 1 | 1 | 1 | 1 | 1 | 1 | 1 | 1 | 1 | 1 |
| **Max** | 6 | 6 | 5 | 5 | 5 | 5 | 5 | 5 | 5 | 5 | 5 | 5 | 5 | 5 | 5 | 5 | 5 | 5 |

**Tabelle 4: Probandenkollektiv A, Rötung, Anwendung Lotion gemäß Beispiel 6**

| **Tag→** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 3 | 3 | 2 | 2 | 2 | 2 | 2 | 2 | 1 | 1 | 1 | 1 | 1 | 1 | 1 | 1 | 1 | 1 |
| **2** | 4 | 3 | 3 | 3 | 3 | 3 | 2 | 2 | 2 | 2 | 2 | 1 | 1 | 1 | 1 | 1 | 1 | 1 |
| **3** | 3 | 3 | 3 | 3 | 2 | 2 | 2 | 2 | 2 | 2 | 2 | 1 | 1 | 1 | 1 | 1 | 1 | 1 |
| **4** | 3 | 3 | 3 | 3 | 3 | 3 | 2 | 2 | 2 | 2 | 2 | 2 | 1 | 1 | 1 | 1 | 1 | 1 |
| **5** | 2 | 2 | 2 | 2 | 2 | 2 | 2 | 2 | 2 | 2 | 2 | 2 | 2 | 2 | 2 | 2 | 2 | 2 |
| **6** | 2 | 2 | 2 | 2 | 2 | 2 | 3 | 3 | 2 | 2 | 3 | 2 | 4 | 2 | 3 | 2 | 2 | 1 |
| **7** | 4 | 3 | 3 | 3 | 3 | 3 | 3 | 3 | 3 | 3 | 2 | 2 | 2 | 2 | 1 | 1 | 1 | 1 |
| **8** | 3 | 3 | 3 | 2 | 2 | 2 | 2 | 2 | 2 | 2 | 1 | 1 | 1 | 1 | 1 | 1 | 1 | 1 |
| **9** | 2 | 2 | 2 | 2 | 2 | 2 | 2 | 2 | 2 | 2 | 2 | 1 | 1 | 1 | 1 | 1 | 1 | 1 |
| **10** | 3 | 3 | 3 | 2 | 2 | 2 | 2 | 2 | 1 | 1 | 1 | 1 | 1 | 1 | 1 | 1 | 1 | 1 |
| **11** | 3 | 2 | 2 | 2 | 2 | 2 | 2 | 1 | 1 | 1 | 1 | 1 | 1 | 1 | 1 | 1 | 1 | 1 |
| **12** | 3 | 3 | 3 | 3 | 3 | 3 | 3 | 3 | 3 | 3 | 3 | 2 | 2 | 2 | 2 | 1 | 1 | 1 |
| **13** | 3 | 3 | 3 | 3 | 3 | 3 | 3 | 2 | 2 | 2 | 2 | 2 | 2 | 1 | 1 | 1 | 1 | 1 |
| **14** | 1 | 1 | 2 | 2 | 2 | 2 | 1 | 1 | 1 | 1 | 1 | 1 | 1 | 1 | 2 | 2 | 1 | 1 |
| **15** | 4 | 4 | 4 | 4 | 4 | 4 | 4 | 4 | 4 | 4 | 4 | 3 | 3 | 3 | 3 | 3 | 3 | 3 |
| **Mittelwert** | 2,6 | 2,7 | 2,7 | 2,5 | 2,5 | 2,5 | 2,3 | 2,2 | 2,0 | 2,0 | 1,9 | 1,6 | 1,6 | 1,4 | 1,5 | 1,3 | 1,3 | 1,2 |
| **Min** | 1 | 1 | 2 | 2 | 2 | 2 | 1 | 1 | 1 | 1 | 1 | 1 | 1 | 1 | 1 | 1 | 1 | 1 |
| **Max** | 4 | 4 | 4 | 4 | 4 | 4 | 4 | 4 | 4 | 4 | 4 | 3 | 4 | 3 | 3 | 3 | 3 | 3 |

**Tabelle 5: Probandenkollektiv A, Trockeneheit, Anwendung Lotion gemäß Beispiel 6**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 3 | 3 | 2 | 2 | 2 | 2 | 2 | 2 | 2 | 2 | 2 | 1 | 1 | 1 | 1 | 1 | 1 | 1 |
| **2** | 3 | 3 | 3 | 3 | 3 | 3 | 2 | 2 | 2 | 2 | 2 | 2 | 2 | 2 | 2 | 1 | 1 | 1 |
| **3** | 3 | 3 | 3 | 3 | 2 | 2 | 2 | 2 | 2 | 2 | 2 | 1 | 1 | 1 | 1 | 1 | 1 | 1 |
| **4** | 3 | 3 | 3 | 3 | 3 | 3 | 2 | 2 | 2 | 2 | 2 | 2 | 1 | 1 | 1 | 1 | 1 | 1 |
| **5** | 3 | 3 | 2 | 2 | 3 | 3 | 3 | 3 | 2 | 2 | 2 | 2 | 2 | 2 | 3 | 3 | 3 | 3 |
| **6** | 2 | 2 | 2 | 3 | 1 | 3 | 1 | 3 | 1 | 3 | 1 | 2 | 3 | 1 | 2 | 1 | 1 | 1 |
| **7** | 4 | 4 | 4 | 3 | 3 | 3 | 3 | 3 | 2 | 2 | 2 | 2 | 2 | 2 | 2 | 1 | 1 | 1 |
| **8** | 2 | 2 | 2 | 2 | 2 | 2 | 2 | 2 | 2 | 2 | 1 | 1 | 1 | 1 | 1 | 1 | 1 | 1 |
| **9** | 3 | 2 | 2 | 3 | 3 | 2 | 2 | 2 | 2 | 2 | 2 | 2 | 2 | 1 | 1 | 1 | 1 | 1 |
| **10** | 3 | 3 | 3 | 3 | 2 | 2 | 2 | 2 | 1 | 1 | 1 | 1 | 1 | 1 | 1 | 1 | 1 | 1 |
| **11** | 3 | 2 | 2 | 2 | 2 | 2 | 2 | 1 | 1 | 1 | 1 | 1 | 1 | 1 | 1 | 1 | 1 | 1 |
| **12** | 3 | 3 | 3 | 3 | 3 | 3 | 3 | 2 | 2 | 2 | 2 | 2 | 2 | 2 | 2 | 1 | 1 | 1 |
| **13** | 4 | 4 | 3 | 3 | 3 | 3 | 3 | 3 | 3 | 2 | 2 | 2 | 1 | 1 | 1 | 1 | 1 | 1 |
| **14** | 1 | 1 | 2 | 2 | 2 | 2 | 1 | 2 | 2 | 2 | 3 | 3 | 2 | 2 | 2 | 2 | 2 | 2 |
| **15** | 4 | 4 | 4 | 4 | 3 | 4 | 4 | 3 | 4 | 4 | 3 | 3 | 3 | 4 | 4 | 4 | 3 | 4 |
| **Mittelwert** | 2,6 | 2,8 | 2,7 | 2,7 | 2,5 | 2,6 | 2,3 | 2,3 | 2,0 | 2,1 | 1,9 | 1,8 | 1,7 | 1,5 | 1,7 | 1,4 | 1,3 | 1,4 |
| **Min** | 1 | 1 | 2 | 2 | 1 | 2 | 1 | 1 | 1 | 1 | 1 | 1 | 1 | 1 | 1 | 1 | 1 | 1 |
| **Max** | 4 | 4 | 4 | 4 | 3 | 4 | 4 | 3 | 4 | 4 | 3 | 3 | 3 | 4 | 4 | 4 | 3 | 4 |

**Tabelle 6: Probandenkollekliv A, Juckreiz, Anwendung Lotion gemäß Beispiel 6**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr.↓** | | | | | | | | | | | | | | | | | | |
| **1** | 3 | 3 | 3 | 2 | 2 | 2 | 2 | 2 | 1 | 1 | 1 | 1 | 1 | 1 | 1 | 1 | 1 | 1 |
| **2** | 4 | 3 | 3 | 3 | 3 | 3 | 3 | 3 | 2 | 2 | 2 | 2 | 2 | 1 | 1 | 1 | 1 | 1 |
| **3** | 3 | 3 | 3 | 3 | 2 | 2 | 2 | 2 | 2 | 2 | 2 | 1 | 1 | 1 | 1 | 1 | 1 | 1 |
| **4** | 3 | 3 | 3 | 3 | 3 | 3 | 2 | 2 | 2 | 2 | 2 | 2 | 1 | 1 | 1 | 1 | 1 | 1 |
| **5** | 2 | 3 | 3 | 3 | 3 | 3 | 3 | 3 | 3 | 3 | 3 | 3 | 2 | 3 | 3 | 3 | 3 | 3 |
| **6** | 2 | 2 | 2 | 1 | 3 | 3 | 1 | 2 | 2 | 2 | 2 | 1 | 2 | 3 | 2 | 2 | 2 | 2 |
| **7** | 4 | 4 | 4 | 3 | 3 | 3 | 3 | 3 | 3 | 3 | 2 | 2 | 2 | 2 | 1 | 1 | 1 | 1 |
| **8** | 3 | 3 | 3 | 3 | 3 | 3 | 2 | 2 | 2 | 2 | 2 | 2 | 1 | 1 | 1 | 1 | 1 | 1 |
| **9** | 3 | 3 | 3 | 3 | 3 | 2 | 2 | 2 | 2 | 2 | 2 | 2 | 2 | 1 | 1 | 1 | 1 | 1 |
| **10** | 2 | 2 | 2 | 2 | 1 | 1 | 1 | 1 | 1 | 1 | 1 | 1 | 1 | 1 | 1 | 1 | 1 | 1 |
| **11** | 3 | 2 | 2 | 2 | 2 | 2 | 2 | 1 | 1 | 1 | 1 | 1 | 1 | 1 | 1 | 1 | 1 | 1 |
| **12** | 3 | 3 | 3 | 3 | 3 | 3 | 3 | 2 | 2 | 2 | 2 | 2 | 2 | 2 | 2 | 1 | 1 | 1 |
| **13** | 3 | 3 | 3 | 3 | 3 | 3 | 3 | 2 | 2 | 2 | 2 | 2 | 1 | 1 | 1 | 1 | 1 | 1 |
| **14** | 1 | 1 | 1 | 1 | 1 | 1 | 1 | 1 | 1 | 1 | 2 | 2 | 1 | 1 | 2 | 2 | 1 | 1 |
| **15** | 4 | 5 | 4 | 4 | 4 | 4 | 4 | 4 | 4 | 4 | 4 | 3 | 3 | 3 | 3 | 3 | 3 | 3 |
| **Mittelwert** | 2,9 | 2,9 | 2,8 | 2,6 | 2,6 | 2,5 | 2,3 | 2,1 | 2,0 | 2,0 | 2,0 | 1,8 | 1,5 | 1,5 | 1,5 | 1,4 | 1,3 | 1,3 |
| **Min** | 1 | 1 | 1 | 1 | 1 | 1 | 1 | 1 | 1 | 1 | 1 | 1 | 1 | 1 | 1 | 1 | 1 | 1 |
| **Max** | 4 | 5 | 4 | 4 | 4 | 4 | 4 | 4 | 4 | 4 | 4 | 3 | 3 | 3 | 3 | 3 | 3 | 3 |

**Tabelle 7: Probandenkollektiv A, Wirkung des Prüfpräparares, Anwendung Lotion gemäß Beispiel 6**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 3 | 3 | 4 | 4 | 5 | 5 | 5 | 5 | 6 | 6 | 6 | 7 | 7 | 7 | 7 | 7 | 7 | 7 |
| **2** | 2 | 3 | 3 | 3 | 3 | 3 | 3 | 3 | 4 | 3 | 3 | 4 | 4 | 5 | 5 | 7 | 7 | 7 |
| **3** | 4 | 4 | 4 | 4 | 4 | 4 | 4 | 4 | 4 | 4 | 4 | 7 | 7 | 7 | 7 | 7 | 7 | 7 |
| **4** | 4 | 4 | 4 | 4 | 4 | 4 | 5 | 5 | 5 | 5 | 5 | 5 | 7 | 7 | 7 | 7 | 7 | 7 |
| **5** | 4 | 4 | 3 | 3 | 3 | 3 | 3 | 3 | 3 | 3 | 3 | 3 | 3 | 3 | 3 | 3 | 3 | 3 |
| **6** | 2 | 2 | 2 | 1 | 3 | 2 | 2 | 2 | 2 | 2 | 3 | 1 | 3 | 3 | 3 | 2 | 2 | 2 |
| **7** | 3 | 3 | 3 | 4 | 4 | 4 | 4 | 4 | 4 | 4 | 4 | 4 | 4 | 5 | 7 | 7 | 7 | 7 |
| **8** | 3 | 3 | 3 | 3 | 3 | 3 | 4 | 4 | 4 | 4 | 4 | 4 | 7 | 7 | 7 | 7 | 7 | 7 |
| **9** | 3 | 3 | 3 | 3 | 3 | 4 | 4 | 4 | 4 | 4 | 4 | 5 | 5 | 6 | 7 | 7 | 7 | 7 |
| **10** | 4 | 4 | 4 | 5 | 6 | 6 | 6 | 6 | 6 | 6 | 6 | 6 | 6 | 6 | 6 | 6 | 6 | 6 |
| **11** | 4 | 4 | 4 | 4 | 4 | 4 | 4 | 5 | 5 | 5 | 5 | 5 | 5 | 5 | 5 | 5 | 5 | 5 |
| **12** | 3 | 3 | 3 | 3 | 3 | 3 | 3 | 4 | 4 | 4 | 4 | 4 | 4 | 5 | 5 | 7 | 7 | 7 |
| **13** | 3 | 3 | 3 | 3 | 3 | 3 | 3 | 3 | 3 | 4 | 4 | 4 | 5 | 5 | 5 | 5 | 5 | 5 |
| **14** | 2 | 2 | 2 | 2 | 2 | 2 | 2 | 2 | 2 | 2 | 2 | 2 | 2 | 2 | 2 | 2 | 2 | 2 |
| **15** | 2 | 3 | 3 | 3 | 3 | 3 | 3 | 3 | 3 | 3 | 4 | 4 | 3 | 3 | 3 | 3 | 3 | 3 |
| **Mittelwert** | 3,1 | 3,2 | 3,2 | 3,3 | 3,5 | 3,5 | 3,7 | 3,8 | 3,9 | 3,9 | 4,1 | 4,3 | 4,8 | 5,1 | 5,3 | 5,5 | 5,5 | 5,5 |
| **Min** | 2 | 2 | 2 | 1 | 2 | 2 | 2 | 2 | 2 | 2 | 2 | 1 | 2 | 2 | 2 | 2 | 2 | 2 |
| **Max** | 4 | 4 | 4 | 5 | 6 | 6 | 6 | 6 | 6 | 6 | 6 | 7 | 7 | 7 | 7 | 7 | 7 | 7 |

**Tabelle 8: Probandenkollektiv B, Schweregrad der Hauterkrankung, Anwendung Lotion gemäß Beispiel 7**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 2 | 2 | 3 | 3 | 3 | 3 | 3 | 3 | 2 | 2 | 2 | 3 | 3 | 3 | 3 | 3 | 3 | 3 |
| **2** | 2 | 2 | 2 | 2 | 2 | 2 | 2 | 2 | 2 | 2 | 2 | 2 | 2 | 2 | 2 | 2 | 2 | 2 |
| **3** | 1 | 1 | 1 | 1 | 1 | 1 | 1 | 1 | 1 | 1 | 1 | 1 | 1 | 1 | 1 | 1 | 1 | 1 |
| **4** | 3 | 3 | 3 | 3 | 3 | 3 | 3 | 3 | 2 | 2 | 2 | 2 | 2 | 2 | 2 | 1 | 1 | 1 |
| **5** | 3 | 3 | 3 | 3 | 3 | 3 | 3 | 2 | 2 | 2 | 2 | 2 | 2 | 2 | 2 | 2 | 1 | 1 |
| **6** | 3 | 3 | 3 | 3 | 3 | 3 | 3 | 3 | 3 | 3 | 3 | 2 | 2 | 2 | 2 | 1 | 1 | 1 |
| **7** | 4 | 4 | 4 | 4 | 4 | 3 | 3 | 3 | 3 | 3 | 3 | 3 | 2 | 2 | 2 | 1 | 1 | 1 |
| **8** | 2 | 3 | 3 | 3 | 3 | 3 | 3 | 3 | 2 | 2 | 2 | 2 | 2 | 2 | 2 | 2 | 2 | 2 |
| **9** | 2 | 2 | 2 | 2 | 2 | 2 | 2 | 2 | 2 | 2 | 2 | 1 | 1 | 1 | 1 | 1 | 1 | 1 |
| **10** | 4 | 4 | 4 | 4 | 4 | 4 | 4 | 3 | 3 | 3 | 3 | 3 | 3 | 3 | 2 | 2 | 2 | 2 |
| **11** | 4 | 4 | 4 | 4 | 4 | 3 | 3 | 3 | 3 | 3 | 3 | 3 | 3 | 3 | 3 | 3 | 3 | 3 |
| **12** | 4 | 4 | 4 | 4 | 4 | 4 | 4 | 4 | 4 | 4 | 3 | 3 | 3 | 3 | 3 | 3 | 3 | 3 |
| **13** | 5 | 5 | 4 | 4 | 4 | 4 | 4 | 4 | 4 | 4 | 4 | 4 | 4 | 4 | 3 | 3 | 3 | 3 |
| **14** | 3 | 3 | 3 | 2 | 2 | 2 | 2 | 2 | 2 | 2 | 2 | 2 | 1 | 1 | 1 | 1 | 1 | 1 |
| **15** | 3 | 3 | 3 | 2 | 2 | 2 | 2 | 2 | 2 | 2 | 2 | 2 | 1 | 1 | 1 | 1 | 1 | 1 |
| **Mittelwert** | 3,0 | 3,1 | 3,1 | 2,9 | 2,9 | 2,8 | 2,8 | 2,7 | 2,5 | 2,5 | 2,4 | 2,3 | 2,1 | 2,1 | 2,0 | 1,8 | 1,7 | 1,7 |
| **Min** | 1 | 1 | 1 | 1 | 1 | 1 | 1 | 1 | 1 | 1 | 1 | 1 | 1 | 1 | 1 | 1 | 1 | 1 |
| **Max** | 5 | 5 | 4 | 4 | 4 | 4 | 4 | 4 | 4 | 4 | 4 | 4 | 4 | 4 | 3 | 3 | 3 | 3 |

**Tabelle 9: Probandenkollekliv B, Rötung, Anwendung Lotion gemäß Beispiel 7**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr.↓** | | | | | | | | | | | | | | | | | | |
| **1** | 2 | 2 | 3 | 3 | 2 | 2 | 2 | 2 | 2 | 22 | 2 | 2 | 2 | 2 | 2 | 3 | 3 | 3 |
| **2** | 3 | 3 | 3 | 3 | 3 | 2 | 2 | 2 | 2 | 2 | 2 | 2 | 2 | 2 | 2 | 2 | 2 | 2 |
| **3** | 1 | 1 | 1 | 1 | 1 | 1 | 1 | 1 | 1 | 1 | 1 | 1 | 1 | 1 | 1 | 1 | 1 | 1 |
| **4** | 3 | 3 | 3 | 3 | 3 | 3 | 3 | 3 | 2 | 2 | 2 | 2 | 2 | 2 | 2 | 1 | 1 | 1 |
| **5** | 3 | 3 | 3 | 3 | 3 | 2 | 2 | 2 | 2 | 2 | 2 | 2 | 2 | 2 | 2 | 2 | 1 | 1 |
| **6** | 3 | 3 | 3 | 3 | 3 | 3 | 3 | 3 | 2 | 2 | 2 | 2 | 2 | 2 | 2 | 1 | 1 | 1 |
| **7** | 4 | 4 | 4 | 4 | 4 | 3 | 3 | 3 | 3 | 3 | 3 | 2 | 2 | 2 | 2 | 1 | 1 | 1 |
| **8** | 1 | 2 | 2 | 3 | 2 | 3 | 2 | 2 | 1 | 1 | 1 | 1 | 1 | 1 | 1 | 1 | 1 | 1 |
| **9** | 2 | 2 | 1 | 1 | 1 | 2 | 2 | 1 | 1 | 1 | 1 | 1 | 1 | 1 | 1 | 1 | 1 | 1 |
| **10** | 4 | 3 | 3 | 3 | 3 | 3 | 3 | 3 | 3 | 3 | 2 | 2 | 2 | 2 | 2 | 2 | 2 | 2 |
| **11** | 3 | 3 | 3 | 3 | 3 | 3 | 3 | 3 | 3 | 3 | 3 | 3 | 3 | 3 | 3 | 3 | 3 | 3 |
| **12** | 4 | 4 | 4 | 4 | 4 | 4 | 4 | 3 | 3 | 3 | 3 | 3 | 3 | 3 | 3 | 3 | 3 | 3 |
| **13** | 5 | 5 | 4 | 4 | 4 | 4 | 4 | 4 | 4 | 4 | 4 | 4 | 4 | 3 | 3 | 3 | 3 | 3 |
| **14** | 3 | 3 | 3 | 2 | 2 | 2 | 2 | 2 | 2 | 2 | 1 | 1 | 1 | 1 | 1 | 1 | 1 | 1 |
| **15** | 2 | 2 | 2 | 2 | 2 | 2 | 2 | 2 | 2 | 2 | 2 | 2 | 1 | 1 | 1 | 1 | 1 | 1 |
| **Mittelwert** | 2,9 | 2,9 | 2,8 | 2,8 | 2,7 | 2,6 | 2,5 | 2,4 | 2,2 | 2,2 | 2,1 | 2,0 | 1,9 | 1,9 | 1,9 | 1,7 | 1,7 | 1,7 |
| **Min** | 1 | 1 | 1 | 1 | 1 | 1 | 1 | 1 | 1 | 1 | 1 | 1 | 1 | 1 | 1 | 1 | 1 | 1 |
| **Max** | 5 | 5 | 4 | 4 | 4 | 4 | 4 | 4 | 4 | 4 | 4 | 4 | 4 | 3 | 3 | 3 | 3 | 3 |

**Tabelle 10: Probandenkollektiv B, Trockenheit, Anwendung Lotion gemäß Beispiel 7**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr.↓** | | | | | | | | | | | | | | | | | | |
| **1** | 2 | 2 | 4 | 3 | 2 | 2 | 2 | 2 | 2 | 2 | 3 | 2 | 3 | 2 | 2 | 3 | 3 | 3 |
| **2** | 3 | 3 | 3 | 2 | 2 | 3 | 3 | 3 | 3 | 3 | 3 | 3 | 2 | 2 | 2 | 2 | 2 | 2 |
| **3** | 1 | 1 | 1 | 1 | 1 | 1 | 1 | 1 | 1 | 1 | 1 | 1 | 1 | 1 | 1 | 1 | 1 | 1 |
| **4** | 3 | 3 | 3 | 3 | 2 | 2 | 2 | 1 | 1 | 1 | 1 | 1 | 1 | 1 | 1 | 1 | 1 | 1 |
| **5** | 3 | 3 | 3 | 2 | 2 | 2 | 2 | 2 | 2 | 2 | 2 | 2 | 2 | 1 | 1 | 1 | 1 | 1 |
| **6** | 3 | 3 | 3 | 3 | 3 | 2 | 2 | 2 | 2 | 2 | 2 | 2 | 2 | 2 | 2 | 1 | 1 | 1 |
| **7** | 3 | 3 | 3 | 3 | 3 | 3 | 3 | 3 | 3 | 3 | 3 | 3 | 2 | 2 | 2 | 1 | 1 | 1 |
| **8** | 3 | 4 | 5 | 5 | 5 | 5 | 4 | 4 | 4 | 4 | 4 | 3 | 4 | 4 | 4 | 3 | 4 | 4 |
| **9** | 2 | 2 | 2 | 2 | 2 | 2 | 2 | 2 | 2 | 1 | 1 | 1 | 1 | 1 | 1 | 1 | 1 | 1 |
| 10 | 4 | 4 | 4 | 4 | 4 | 3 | 3 | 3 | 3 | 3 | 3 | 3 | 2 | 2 | 2 | 2 | 2 | 2 |
| **11** | 3 | 3 | 3 | 3 | 3 | 3 | 3 | 3 | 3 | 3 | 3 | 3 | 3 | 3 | 3 | 3 | 3 | 3 |
| **12** | 4 | 4 | 4 | 4 | 4 | 4 | 4 | 3 | 3 | 3 | 3 | 3 | 3 | 3 | 3 | 3 | 3 | 3 |
| **13** | 4 | 4 | 4 | 4 | 4 | 4 | 4 | 4 | 4 | 4 | 4 | 4 | 4 | 3 | 3 | 3 | 3 | 3 |
| **14** | 2 | 2 | 2 | 2 | 2 | 2 | 2 | 2 | 2 | 1 | 1 | 1 | 1 | 1 | 1 | 1 | 1 | 1 |
| **15** | 2 | 2 | 2 | 2 | 2 | 2 | 2 | 2 | 2 | 2 | 2 | 2 | 1 | 1 | 1 | 1 | 1 | 1 |
| **Mittelwert** | 2,8 | 2,9 | 3,1 | 2,9 | 2,7 | 2,7 | 2,6 | 2,5 | 2,5 | 2,3 | 2,4 | 2,3 | 2,1 | 1,9 | 1,9 | 1,8 | 1,9 | 1,9 |
| **Min** | 1 | 1 | 1 | 1 | 1 | 1 | 1 | 1 | 1 | 1 | 1 | 1 | 1 | 1 | 1 | 1 | 1 | 1 |
| **Max** | 4 | 4 | 5 | 5 | 5 | 5 | 4 | 4 | 4 | 4 | 4 | 4 | 4 | 4 | 4 | 3 | 4 | 4 |

**Tabelle 11: Probandenkollektiv B, Juckreiz, Anwendung Lotion gemäß Beispiel 7**

| **Tag→** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 1 | 1 | 2 | 3 | 3 | 2 | 2 | 2 | 2 | 2 | 3 | 2 | 2 | 2 | 2 | 3 | 3 | 3 |
| **2** | 2 | 2 | 3 | 3 | 2 | 2 | 3 | 3 | 3 | 3 | 2 | 2 | 2 | 2 | 2 | 2 | 2 | 2 |
| **3** | 1 | 1 | 1 | 1 | 1 | 1 | 1 | 1 | 1 | 1 | 1 | 1 | 1 | 1 | 1 | 1 | 1 | 1 |
| **4** | 3 | 3 | 3 | 3 | 3 | 3 | 3 | 3 | 2 | 2 | 2 | 2 | 1 | 1 | 1 | 1 | 1 | 1 |
| **5** | 3 | 3 | 3 | 3 | 3 | 3 | 3 | 2 | 2 | 2 | 2 | 2 | 2 | 1 | 1 | 1 | 1 | 1 |
| **6** | 3 | 3 | 3 | 3 | 3 | 3 | 3 | 3 | 3 | 3 | 2 | 2 | 2 | 2 | 2 | 1 | 1 | 1 |
| **7** | 4 | 4 | 4 | 3 | 3 | 3 | 3 | 3 | 3 | 3 | 3 | 3 | 2 | 2 | 2 | 1 | 1 | 1 |
| **8** | 1 | 2 | 2 | 3 | 3 | 4 | 2 | 2 | 1 | 1 | 1 | 1 | 1 | 1 | 1 | 1 | 1 | 1 |
| **9** | 2 | 2 | 2 | 2 | 2 | 2 | 2 | 2 | 2 | 2 | 2 | 1 | 1 | 1 | 1 | 1 | 1 | 1 |
| **10** | 4 | 4 | 4 | 4 | 4 | 4 | 4 | 3 | 3 | 3 | 3 | 3 | 3 | 3 | 2 | 2 | 2 | 2 |
| **11** | 4 | 4 | 4 | 4 | 3 | 3 | 3 | 3 | 3 | 3 | 3 | 3 | 3 | 3 | 3 | 3 | 3 | 3 |
| **12** | 4 | 4 | 4 | 4 | 4 | 4 | 4 | 4 | 4 | 4 | 3 | 3 | 3 | 3 | 3 | 3 | 3 | 3 |
| 13 | 4 | 4 | 4 | 4 | 4 | 4 | 4 | 4 | 4 | 4 | 4 | 4 | 4 | 3 | 3 | 3 | 3 | 3 |
| **14** | 3 | 3 | 2 | 2 | 2 | 2 | 2 | 2 | 2 | 2 | 2 | 2 | 1 | 1 | 1 | 1 | 1 | 1 |
| **15** | 3 | 3 | 2 | 2 | 2 | 2 | 2 | 2 | 2 | 2 | 2 | 2 | 1 | 1 | 1 | 1 | 1 | 1 |
| **Mittelwert** | 2,8 | 2,9 | 2,9 | 2,9 | 2,8 | 2,8 | 2,7 | 2,6 | 2,5 | 2,5 | 2,3 | 2,2 | 1,9 | 1,8 | 1,7 | 1,7 | 1,7 | 1,7 |
| **Min** | 1 | 1 | 1 | 1 | 1 | 1 | 1 | 1 | 1 | 1 | 1 | 1 | 1 | 1 | 1 | 1 | 1 | 1 |
| **Max** | 4 | 4 | 4 | 4 | 4 | 4 | 4 | 4 | 4 | 4 | 4 | 4 | 4 | 3 | 3 | 3 | 3 | 3 |

**Tabelle 12: Probandenkollektiv B, Wirkung des Prüfpräparates, Anwendung Lotion gemäß Beispiel 7**

| **Tag →** | **11** | **12** | **13** | **14** | **15** | **16** | **17** | **18** | **19** | **20** | **21** | **22** | **23** | **24** | **25** | **26** | **27** | **28** |
|---|---|---|---|---|---|---|---|---|---|---|---|---|---|---|---|---|---|---|
| **Nr. ↓** | | | | | | | | | | | | | | | | | | |
| **1** | 4 | 4 | 3 | 4 | 4 | 5 | 5 | 5 | 5 | 5 | 4 | 4 | 4 | 5 | 5 | 2 | 4 | 5 |
| **2** | 3 | 3 | 3 | 2 | 2 | 2 | 2 | 2 | 2 | 2 | 2 | 2 | 2 | 2 | 2 | 2 | 2 | 2 |
| **3** | 6 | 6 | 6 | 6 | 6 | 6 | 6 | 6 | 6 | 6 | 6 | 6 | 6 | 6 | 6 | 6 | 6 | 6 |
| **4** | 3 | 3 | 3 | 3 | 3 | 3 | 3 | 3 | 4 | 4 | 4 | 4 | 5 | 5 | 5 | 6 | 6 | 6 |
| **5** | 3 | 3 | 3 | 3 | 3 | 3 | 3 | 4 | 4 | 4 | 4 | 4 | 4 | 5 | 5 | 5 | 6 | 6 |
| **6** | 4 | 4 | 4 | 4 | 4 | 3 | 4 | 4 | 4 | 4 | 4 | 5 | 5 | 5 | 5 | 6 | 7 | 7 |
| **7** | 3 | 3 | 3 | 4 | 4 | 5 | 5 | 5 | 5 | 5 | 5 | 5 | 6 | 6 | 6 | 7 | 7 | 7 |
| **8** | 2 | 3 | 3 | 4 | 3 | 4 | 3 | 3 | 2 | 2 | 2 | 2 | 2 | 2 | 2 | 2 | 2 | 2 |
| **9** | 4 | 4 | 5 | 5 | 5 | 4 | 4 | 5 | 5 | 5 | 5 | 6 | 6 | 6 | 6 | 6 | 6 | 6 |
| **10** | 3 | 3 | 3 | 3 | 3 | 3 | 3 | 4 | 4 | 4 | 4 | 4 | 4 | 4 | 4 | 4 | 4 | 4 |
| **11** | 2 | 2 | 2 | 2 | 3 | 3 | 3 | 3 | 3 | 3 | 3 | 3 | 3 | 3 | 3 | 3 | 3 | 3 |
| **12** | 4 | 4 | 4 | 4 | 4 | 4 | 4 | 4 | 4 | 5 | 5 | 5 | 5 | 5 | 5 | 5 | 5 | 5 |
| **13** | 3 | 3 | 4 | 4 | 4 | 4 | 4 | 4 | 4 | 4 | 4 | 4 | 4 | 5 | 5 | 5 | 5 | 5 |
| **14** | 3 | 3 | 4 | 4 | 4 | 4 | 4 | 4 | 4 | 4 | 4 | 4 | 5 | 5 | 5 | 5 | 5 | 5 |
| **15** | 3 | 3 | 4 | 4 | 4 | 4 | 4 | 4 | 4 | 4 | 4 | 4 | 5 | 5 | 5 | 5 | 5 | 5 |
| **Mittelwert** | 3,3 | 3,4 | 3,6 | 3,7 | 3,7 | 3,8 | 3,8 | 4,0 | 4,0 | 4,1 | 4,0 | 4,1 | 4,4 | 4,6 | 4,6 | 4,6 | 4,9 | 4,9 |
| **Min** | 2 | 2 | 2 | 2 | 2 | 2 | 2 | 2 | 2 | 2 | 2 | 2 | 2 | 2 | 2 | 2 | 2 | 2 |
| **Max** | 6 | 6 | 6 | 6 | 6 | 6 | 6 | 6 | 6 | 6 | 6 | 6 | 6 | 6 | 6 | 7 | 7 | 7 |

**Tabelle 13: Atopie-Score, Probandenkollektiv A, 18-tägige Anwendung der Lotion gemäß Beispiel 6**

| **Probandennr.** | **Eingangswert** | **Nach 18 Tagen** | **Veränderung absolut** | **Veränderung prozentual** |
|---|---|---|---|---|
| 1. | 36,7 | 0 | -36,7 | -100,00 |
| 2. | 29,8 | 0 | -29,8 | -100,00 |
| 3. | 32,2 | 0 | -32,2 | -100,00 |
| 4. | 25,5 | 0 | -25,5 | -100,00 |
| 5. | 36,9 | 25,7 | -11,2 | -30,35 |
| 6. | 24,9 | 21,7 | -3,2 | -12,85 |
| 7. | 24,9 | 0 | -24,9 | -100,00 |
| 8. | 25,3 | 0 | -25,3 | -100,00 |
| 9. | 25,3 | 0 | -25,3 | -100,00 |
| 10. | 13,3 | 0 | -13,3 | -100,00 |
| 11. | 22,7 | 11,6 | -11,1 | -48,90 |
| 12. | 27,8 | 0 | -27,8 | -100,00 |
| 13. | 23,8 | 4,8 | -19 | -79,83 |
| 14. | 29 | 5,3 | -23,7 | -81,72 |
| 15. | 29 | 26,8 | -2,2 | -7,59 |
| | | | | |
| **Durchschnitt** | **27,14** | **6,39** | **-20,75** | **-76,44** |
| | | | | |
| **Minimum** | 13,30 | 0,00 | -36,70 | -100,00 |
| **Maximum** | 36,90 | 26,80 | -2,20 | -7,59 |

**Tabelle 14: Atopie-Score, Probandenkollektiv B, 18-tägige Anwendung der Lotion gemäß Beispiel 3**

| **Probandennr.** | **Nach Vorbehandlung** | **Nach 18 Tagen** | **Veränderung absolut** | **Veränderung prozentual** |
|---|---|---|---|---|
| 1. | 34,4 | 30,9 | -3,5 | -10,17 |
| 2. | 11,3 | 0 | -11,3 | -100,00 |
| 3. | 22,2 | 0 | -22,2 | -100,00 |
| 4. | 13,6 | 0 | -13,6 | -100,00 |
| 5. | 39,3 | 0 | -39,3 | -100,00 |
| 6. | 13,1 | 0 | -13,1 | -100,00 |
| 7. | 26,8 | 0 | -26,8 | -100,00 |
| 8. | 29,9 | 4,3 | -25,6 | -85,62 |
| 9. | 14 | 13,8 | -0,2 | -1,43 |
| 10. | 5,3 | 0 | -5,3 | -100,00 |
| 11. | 18,6 | 18,4 | -0,2 | -1,08 |
| 12. | 18,3 | 18,3 | 0 | 0,00 |
| 13. | 20,2 | 19,1 | -1,1 | -5,45 |
| 14. | 12,1 | 0 | -12,1 | -100,00 |
| 15. | 7,6 | 0 | -7,6 | -100,00 |
| | | | | |
| **Durchschnitt** | **19,11** | **6,99** | **-12,13** | **-63,45** |
| | | | | |
| **Minimum** | 5,30 | 0,00 | -39,30 | -100,00 |
| **Maximum** | 39,30 | 30,90 | 0,00 | 0,00 |

**Tabelle 15: Atopie-Score des gesamten Pflegesystems**

| **Probandennr.** | **Eingangswert** | **Wert nach 28 Tagen** | **Veränderung absolut** | **Veränderung prozentual** |
|---|---|---|---|---|
| 1. | 48,2 | 0 | -48,2 | -100,00 |
| 2. | 41,6 | 0 | -41,6 | -100,00 |
| 3. | 35,7 | 0 | -35,7 | -100,00 |
| 4. | 39 | 0 | -39 | -100,00 |
| 5. | 37,9 | 25,7 | -12,2 | -32,19 |
| 6. | 37,6 | 21,7 | -15,9 | -42,29 |
| 7. | 39,4 | 0 | -39,4 | -100,00 |
| 8. | 40,5 | 0 | -40,5 | -100,00 |
| 9. | 36,8 | 0 | -36,8 | -100,00 |
| 10. | 28,6 | 0 | -28,6 | -100,00 |
| 11. | 38,2 | 11,6 | -26,6 | -69,63 |
| 12. | 40,3 | 0 | -40,3 | -100,00 |
| 13. | 38,8 | 4,8 | -34 | -87,63 |
| 14. | 41,7 | 5,3 | -36,4 | -87,29 |
| 15. | 40,1 | 26,8 | -13,3 | -33,17 |
| 16. | 47,5 | 30,9 | -16,6 | -34,95 |
| 17. | 23,7 | 0 | -23,7 | -100,00 |
| 18. | 43,5 | 0 | -43,5 | -100,00 |
| 19. | 41,1 | 0 | -41,1 | -100,00 |
| 20. | 40,5 | 0 | -40,5 | -100,00 |
| 21. | 33,2 | 0 | -33,2 | -100,00 |
| 22. | 42,8 | 0 | -42,8 | -100,00 |
| 23. | 46,9 | 4,3 | -42,6 | -90,83 |
| 24. | 35 | 13,8 | -21,2 | -60,57 |
| 25. | 21,5 | 0 | -21,5 | -100,00 |
| 26. | 39,7 | 18,4 | -21,3 | -53,65 |
| 27. | 40,2 | 18,3 | -21,9 | -54,48 |
| 28. | 47,6 | 19,1 | -28,5 | -59,87 |
| 29. | 40,1 | 0 | -40,1 | -100,00 |
| 30. | 25,3 | 0 | -25,3 | -100,00 |
| | | | | |
| **Durchschnitt** | **38,43** | **6,69** | **-31,74** | **-82,59** |
| | | | | |
| **Minimum** | 21,50 | 0,00 | -48,20 | -100,00 |
| **Maximum** | 48,20 | 30,90 | -12,20 | -32,19 |

## Patentansprüche

1. Zusammensetzung, umfassend Silberpartikel einer mittleren Partikelgröße von 1-100 µm und Nachtkerzenöl.

2. Zusammensetzung gemäß Anspruch 1, wobei die Silberpartikel einen mittleren Partikeldurchmesser von 2 bis 20 µm, bevorzugt 2 bis 5 µm aufweisen.

3. Zusammensetzung gemäß einem der Ansprüche 1 oder 2, wobei die Silberpartikel porös sind.

4. Zusammensetzung gemäß einem der Ansprüche 1-3, wobei die Silberpartikel in der Zusammensetzung in Mengen von 0,001 Gew.% - 10 Gew.-%, bevorzugt von 0,1 Gew.-% - 5 Gew.-%, besonders bevorzugt von 0,1 Gew.-% - 1 Gew.-% sowie insbesondere von 0,1 Gew.-% - 0,5 Gew.-% vorliegen.

5. Zusammensetzung gemäß einem der Ansprüche 1-4, wobei das Nachtkerzenöl in der Zusammensetzung in Mengen von 0,1 Gew.-% - 30 Gew.-%, bevorzugt von 1 Gew.-% - 10 Gew.-% vorliegt.

6. Zusammensetzung gemäß einem der Ansprüche 1-5, wobei die Zusammensetzung als alkoholische O/W-Emulsion vorliegt.

7. Zusammensetzung gemäß einem der Ansprüche 1-6, wobei die Zusammensetzung frei von PEG-Emulgatoren und/oder Konservierungsstoffen und/oder Parfüm ist.

8. Zusammensetzung gemäß einem der Ansprüche 1-7, wobei die Zusammensetzung als Lotion, Creme, Emulsion, Salbe, Gel, Puder oder Suspension formuliert ist.

9. Verwendung einer Zusammensetzung gemäß einem der Ansprüche 1-8 zur Pflege trockener Hautzustände.

10. Verwendung gemäß Anspruch 9, wobei die Zusammensetzung topisch auf die trockenen Hautpartien aufgetragen wird.

11. Verwendung gemäß Anspruch 9 oder 10, wobei die Pflege der trockenen Haut in einer Minderung der Hautrötung und/oder der Schuppung besteht.

12. Verwendung von Silberpartikeln einer mittleren Partikelgröße von 1 bis 100 µm in Kombination mit Nachtkerzenöl zur Herstellung einer pharmazeutischen Zusammensetzung zur Behandlung der Neurodermitis.

13. Verwendung gemäß Anspruch 12, wobei die Silberpartikel einen mittleren Partikeldurchmesser von 2 bis 20 µm, bevorzugt 2 bis 5 µm aufweisen.

14. Verwendung nach einem der Ansprüche 12 oder 13, wobei die Silberpartikel porös sind.

15. Verwendung gemäß einem der Ansprüche 12-14, wobei die Zusammensetzung topisch auf die von Neurodermitis betroffenen Hautpartien aufzutragen ist.

16. Verwendung gemäß Anspruch 15, wobei die Silberpartikel in der Zusammensetzung in Mengen von 0,001 Gew.-% bis 10 Gew.-%, bevorzugt von 0,01 Gew.-% - 5 Gew.-%, besonders bevorzugt von 0,1 Gew.-% - 1 Gew.-% sowie insbesondere von 0,1 Gew.-% - 0,5 Gew.-% vorliegen.

17. Verwendung gemäß Anspruch 15 oder 16, wobei das Nachtkerzenöl in der Zusammensetzung in Mengen von 0,1 Gew.-% bis 30 Gew.-%, bevorzugt von 0,1 Gew.- % - 10 Gew.-% vorliegt.

18. Verwendung gemäß einem der Ansprüche 15-17, wobei die Zusammensetzung als alkoholische O/W-Emulsion vorliegt.

19. Verwendung nach einem der Ansprüche 15-18, wobei die Zusammensetzung frei von PEG-Emulgatoren und/oder Konservierungsstoffen und/oder Parfüm ist.

20. Verwendung gemäß einem der Ansprüche 15-19, wobei die Zusammensetzung als Lotion, Paste, Salbe, Creme, Emulsion, Gel, Puder oder Suspension formuliert ist.

21. Verwendung gemäß einem der Ansprüche 12-20, wobei die Behandlung der Neurodermitis in einer Minderung der Ödem/Papeln-Bildung der Haut und/oder der Hautrötung und/oder der Erythembildung und/oder der Krustenbildung und/oder Excoriation und/oder der Lichenifikation und/oder des Juckreizes und/oder der Xerosis und/oder der Schuppung besteht und/oder die Behandlung der Neurodermitis in einer Minderung des Schlafmangels besteht.

22. Verwendung gemäß einem der Ansprüche 12-21, wobei durch die topische Anwendung der Zusammensetzung der durchschnittliche Atopie-Wert um mindestens 40 %, bevorzugt 60 %, besonders bevorzugt 70 % und insbesondere 80 % gesenkt wird.

## Claims

1. Composition comprising silver particles with a mean particle size of from 1 to 100 µm and evening primrose oll.

2. Composition according to claim 1, wherein the silver particles have a mean particle diameter of from 2 to 20 µm, preferably from 2 to 5 µm.

3. Composition according to claim 1 or 2, wherein the silver particles are porous.

4. Composition according to any one of claims 1 - 3, wherein the composition comprises silver particles in an amount of from 0.001 wt.% to 10 wt.%, preferably from 0.1 wt.% % to 5 wt.%, more preferred from 0.1 wt.% to 1 wt.%, and most preferred from D.1 wt.% to 0.5wt%.

5. Composition according to any one of claims 1 -4, wherein the composition comprises the evening primrose oil in an amount of from 0.1 wt.% to 30 wt %, preferably from 1 wt.% to 10 wt.%.

6. Composition according to any one of claims 1 - 5, wherein the composition is an alcoholic O/W emulsion.

7. Composition according to any one of claims 1 - 6, wherein the composition is free of PEG emulsifiers and/or preservatives and/or perfume.

8. Composition according to any one of claims 1 - 7, wherein the composition is formulated as a lotion, cream, emulsion, ointment, gel, powder or suspension.

9. Use of a composition according to any one of claims 1-8 for care of dry skin conditions.

10. Use according to claim 9, wherein the composition is topically applied to dry skin areas.

11. Use according to claim 9 or 10, wherein providing care to dry skin consists of reducing skin redness and/or flake formation.

12. Use of silver particles of a mean particle size of from 1 to 100 µm in combination with evening primrose oil for manufacturing a pharmaceutical formulation for the treatment of neurodermitis.

13. Use according to claim 12, wherein the silver particles have a mean particle diameter ranging from 2 to 20 µm, preferably from 2 to 5 µm.

14. Use according to claim 12 or 13, wherein the silver particles are porous.

15. Use according to any one of claims 12-14, wherein the composition is to be applied topically to skin areas affected by neurodermitis.

16. Use according to claim 15, wherein the composition contains silver particles in an amount of from 0.001 wt. % to 10 wt.%, preferably from 0.01 wt.% to 5 wt.%, very preferred from 0.1 wt.% to 1 wt.%, and most preferred from 0.1 wt.% to 0.5 wt.%,

17. Use according to claim 15 or 16, wherein the composition comprises evening primrose oil in an amount of from 0,1 wt,% to 30 wt.%, preferably from 1 wt.% to 10 wt.%.

18. Use according to any one of claims 15-17, wherein the composition is an alcoholic O/W emulsion.

19. Use according to any one of claims 15-18, wherein the composition is free of PEG emulsifiers and/or preservatives and/or perfume.

20. Use according to any one of claims 15-19, wherein the composition is formulated as a lotion, paste, ointment, cream, emulsion, gel, powder or suspension.

21. Use according to any one of claims 12-20, wherein the treatment of neurodermitis consists of a reduction of edema/papule formation of the skin and/or skin redness and/or the formation of erythema and/or crust formation and/or excoriatlon and/or lichenification and/or pruritus and/or xerosis and/or flake formation, and/or the treatment of neurodermitis consists of a reduction of sleep deficit.

22. Use according to any one of claims 12-21, wherein the average atopy value is decreased by at least 40%, preferably by 60%, more preferred by 70%, and most preferred by 80%.

## Revendications

1. Composition comprenant des particules d'argent ayant une taille de particule moyenne comprise entre 1 et 100 µm et de l'huile d'onagre.

2. Composition selon la revendication 1, dans laquelle les particules d'argent présentent un diamètre de particule moyen compris entre 2 et 20 µm, de préférence entre 2 et 5 µm.

3. Composition selon l'une des revendications 1 et 2, les particules d'argent étant poreuses.

4. Composition selon l'une des revendications 1 à 3, les particules d'argent entrant dans la composition à hauteur de 0,001 % en poids à 10 % en poids, de préférence de 0,1 % en poids à 5 % en poids, de manière particulièrement préférée de 0,1 % en poids à 1 % en poids et en particulier de 0,1 % en poids à 0,5 % en poids.

5. Composition selon l'une des revendications 1 à 4, l'huile d'onagre entrant dans la composition à hauteur de 0,1 % en poids à 30 % en poids, de préférence de 1 % en poids à 10 % en poids.

6. Composition selon l'une des revendications 1 à 5, la composition se présentant sous la forme d'une émulsion H/E alcoolique.

7. Composition selon l'une des revendications 1 à 6, la composition étant exempte d'émulsifiants PEG et/ou de conservateurs et/ou de parfum.

8. Composition selon l'une des revendications 1 à 7, la composition étant formulée sous la forme d'une lotion, d'une crème, d'une émulsion, d'une pommade, d'un gel, d'une poudre ou d'une suspension.

9. Utilisation d'une composition selon l'une des revendications 1 à 8 pour soigner la sécheresse de la peau.

10. Utilisation selon la revendication 9, la composition étant appliquée localement sur les parties sèches de la peau.

11. Utilisation selon la revendication 9 ou la revendication 10, soigner la peau sèche consistant à diminuer les rougeurs cutanées et/ou la desquamation.

12. Utilisation de particules d'argent ayant une taille de particule moyenne comprise entre 1 et 100 µm en combinaison avec de l'huile d'onagre pour la fabrication d'une composition pharmaceutique destinée au traitement de la neurodermatite.

13. Utilisation selon la revendication 12, les particules d'argent présentant un diamètre de particule moyen compris entre 2 et 20 µm, de préférence entre 2 et 5 µm.

14. Utilisation selon l'une des revendications 12 et 13, les particules d'argent étant poreuses.

15. Utilisation selon l'une des revendications 12 à 14, la composition devant être appliquée localement sur les parties de peau affectées par la neurodermatite.

16. Utilisation selon la revendication 15, les particules d'argent entrant dans la composition à hauteur de 0,001 % en poids à 10 % en poids, de préférence de 0,01 % en poids à 5 % en poids, de manière particulièrement préférée de 0,1 % en poids à 1 % en poids et en particulier de 0,1 % en poids à 0,5 % en poids.

17. Utilisation selon la revendication 15 ou la revendication 16, l'huile d'onagre entrant dans la composition à hauteur de 0,1 % en poids à 30 % en poids, de préférence de 0,1 % en poids à 10 % en poids.

18. Utilisation selon l'une des revendications 15 à 17, la composition se présentant sous la forme d'une émulsion H/E alcoolique.

19. Utilisation selon l'une des revendications 15 à 18, la composition étant exemple d'émulsifiants PEG et/ou de conservateurs et/ou de parfum.

20. Utilisation selon l'une des revendications 15 à 19, la composition étant formulée sous la forme d'une lotion, d'une pâte, d'une pommade, d'une crème, d'une émulsion, d'un gel, d'une poudre ou d'une suspension.

21. Utilisation selon l'une des revendications 12 à 20, le traitement de la neurodermatite consistant en une diminution de la formation d'oedèmes/papules cutanés et/ou des rougeurs cutanées et/ou de la formation d'érythèmes et/ou de la formation de croûtes et/ou de l'excoriation et/ou de la lichénification et/ou du prurit et/ou de la xérose et/ou de la desquamation et/ou le traitement de la neurodermatite consistant en une diminution du manque de sommeil.

22. Utilisation selon l'une des revendications 12 à 21, le score atopique moyen étant diminué, par l'application locale de la composition, d'au moins 40 %, de préférence de 60 %, de manière particulièrement préférée de 70 % et en particulier de 80 %.
